(19) Europäisches Patentamt
European Patent Office
Office européen des brevets

(11) Publication number : **0 324 717 B1**

# (12) EUROPEAN PATENT SPECIFICATION

(45) Date of publication of patent specification : **29.04.92 Bulletin 92/18**

(51) Int. Cl.$^5$ : **C09B 23/04,** G03C 1/16, // C07D417/06

(21) Application number : **89810004.5**

(22) Date of filing : **04.01.89**

## (54) Negative-working silver halide emulsion containing certain monomethine compounds.

(30) Priority : **13.01.88 GB 8800662**

(43) Date of publication of application : **19.07.89 Bulletin 89/29**

(45) Publication of the grant of the patent : **29.04.92 Bulletin 92/18**

(84) Designated Contracting States : **BE CH DE FR GB IT LI NL**

(56) References cited :
**US-A- 3 156 685**
**US-A- 4 769 316**
**CHEMICAL ABSTRACTS, vol. 97, no. 12, 20th September 1982, page 92, abstract no. 93957h, Columbus, Ohio, US; & SU-A-910 700 (ALL-UNION SCIENTIFIC-RESEARCH INSTITUTE OF THE PHOTOGRAPHIC-CHEMICAL INDUSTRY) 07-03-1982**

(73) Proprietor : **ILFORD Limited**
**Town Lane**
**Mobberley Knutsford Cheshire WA16 7HA (GB)**

(72) Inventor : **Ficken, Geoffrey Ernest**
**3 Kings Road**
**Wilmslow Cheshire 3K9 5PZ (GB)**
Inventor : **Edwards, Douglas James**
**37 Alderley Road**
**Thelwall Warrington Cheshire (GB)**
Inventor : **Mowforth, Clive William**
**32 Wingfield Avenue**
**Wilmslow Cheshire (GB)**
Inventor : **Maternaghan, Trevor James**
**12 Tabley Close**
**Knutsford Cheshire (GB)**
Inventor : **Steiger, Rolf, Dr.**
**Russille**
**CH-1724 Praroman (CH)**
Inventor : **Dolden, Victor William**
**17 Windsor Avenue**
**Wilmslow Cheshire SK9 5HE (GB)**

**EP 0 324 717 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the use of certain monomethine compounds in silver halide photographic material of the negative working type.

Monomethine cyanine dyes have found great use in silver halide photographic materials.

For example in United States Patent Specification 4769316 there is described a restraining method for restraining the formation of a re-reversal negative image comprising incorporating a cyanine dye or a merocyanine dye which is electron donative and is adsorbable on silver halide in at least one hydrophilic colloid layer of an internal latent image type direct positive silver halide photographic material as a re-reversal restrainer.

A large number of cyanine or merocyanine dyes are listed in USP 4769316 as being of use as restrainers for positive silver halide emulsions.

We have found that one of the classes of monomethine compounds listed in USP 4769316 has particular utility in negative working silver halide emulsions.

Therefore, according to the present invention there is provided a photographic silver halide negative working emulsion which comprises a monomethine compound of the general formula I:-

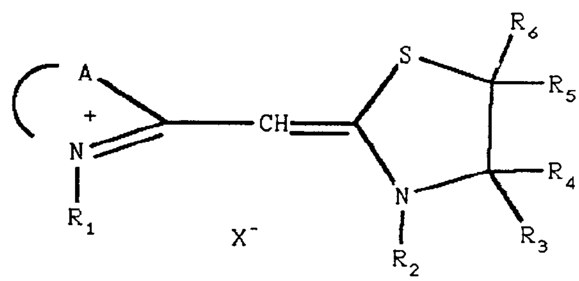

. . I

where A represents the atoms necessary to complete a benzoxazole or naphthoxazole ring which is optionally substituted, $R_1$ and $R_2$ are each alkyl, hydroxyalkyl or alkoxy alkyl groups wherein the alkyl moiety comprises 1 to 4 carbon atoms, $R_3$, $R_4$, $R_5$ and $R_5$ are each hydrogen atoms or alkyl groups having 1 to 4 carbon atoms or phenyl groups, and X is an anion.

Referring to formula I, examples of optional substitution in A include halogen atoms or alkyl or alkoxy group groups showing 1 to 4 carbon atoms or phenyl groups.

The preferred monomethine compounds of formula I for use in the silver halide emulsions are those wherein A represents the atoms necessary to complete a benzoxazole or naphthoxazole ring which is unsubstituted or substituted by alkyl or alkoxy each having 1 to 4 carbon atoms, halogen or phenyl; $R_1$ and $R_2$ are each alkyl or alkoxy alkyl wherein the alkyl moiety comprises 1 to 4 carbon atoms, $R_3$, $R_4$, $R_5$ and $R_5$ are each hydrogen, alkyl having 1 to 4 carbon atoms or phenyl and $X^-$ is $ClO_4^-$, $BF_4^-$ or a halide.

Most preferably $R_1$ and $R_2$ are each methyl groups.

Particularly preferred monomethine compounds for use in silver halide emulsions are compounds A to N as set forth in the Examples which follow.

The compounds of formula I may be prepared by reacting a compound of formula II:-

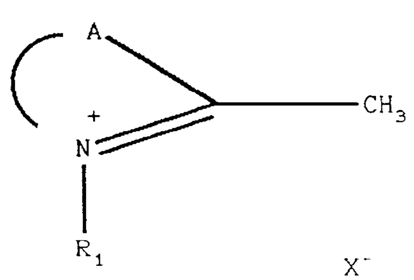

with a compound of formula III:-

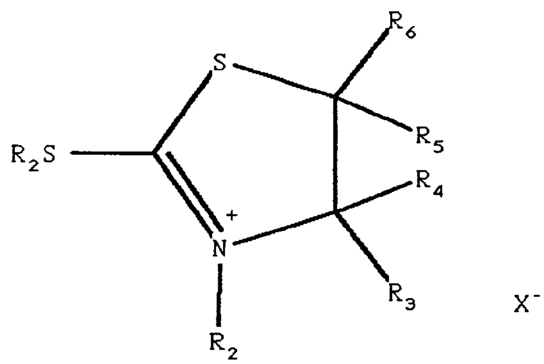

where A, $R_1$-$R_6$ and X have the meanings assigned to them above, at an elevated temperature in the presence of a basic condensing agent. The preferred temperature range is from 80 to 140°C.

Suitable condensing agents are pyridine and triethylamine for this reaction and those which follow.

Alternatively when $R_1$=$R_2$ compounds of formula I may be prepared by heating together compound IV:-

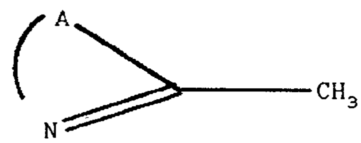

with compound V:-

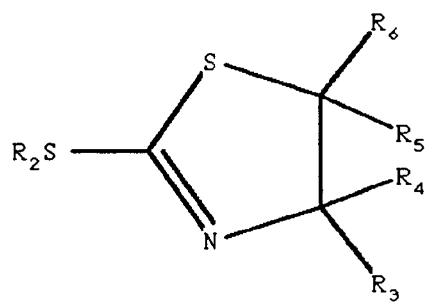

where $R_2$-$R_6$ have the meanings assigned to them above with an excess of a sulphonicester of formula $R_7$-$SO_3$-$R_2$ where $R_2$ has the meaning assigned to it above and $R_7$ is an alkyl or aryl group, followed by heating in the presence of a basic condensing agent.

Compounds of formula I may alternatively be prepared by reaction of compound VI:-

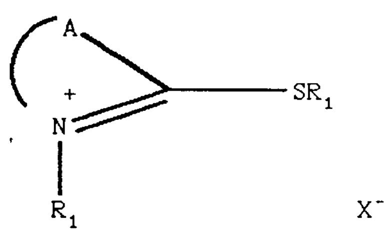

with a compound VII:-

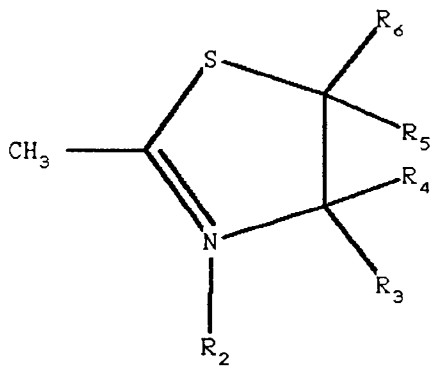

in the presence of a basic condensing agent wherein A, $R_1$-$R_6$ and X are as defined above.

When $R_1$=$R_2$, compounds of formula I may be prepared by heating together a compound of formula VIII:-

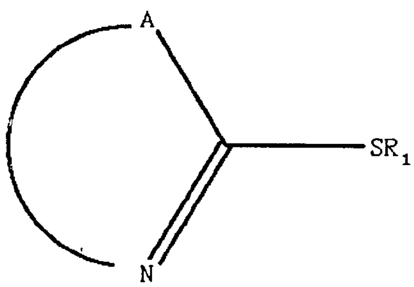

and a compound of formula IX:-

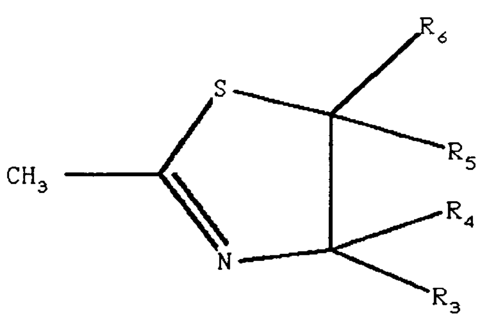

with an excess of a sulphonic ester $R_7SO_3R_2$, followed by heating, preferably to reflux, in the presence of a basic condensing agent.

The most suitable basic condensing agent is pyridine for this reaction.

Compounds of formula I absorb in the UV part of the spectrum but exhibit a useful effect when added to the silver halide emulsion as they improve the overall white light speed of the emulsion. They also appear to improve the inherent sensitivity of the silver halide grains and decrease reciprocity failure at long exposure times.

The compounds of formula I are of particular use to improve the inherent sensitivity of silver halide emulsions wherein the silver halide crystals are of the tabular habit, that is to say wherein the aspect ratio of the crystals is greater than 5:1. This is demonstrated in Example I which follows.

The silver halide emulsions of the present invention which comprise compounds of formula I also have an improved inherent blue light sensitivity and Example 2 illustrates this. Example 3 shows the effect when a compound of formula I is present in a silver halide emulsion which has been optically sensitised with a green sensitising dye. This Example shows that reciprocity failure is reduced at long exposure times.

Preferably the silver halide emulsion of the present invention comprises from 50mg per mole of Ag to 700mg per mole of Ag of the compound of formula I.

When the silver halide emulsions of the present invention have tabular silver halide grains preferably the compound of formula I is present in the range of from 100mg per mole of Ag to 400mg per mole of Ag.

When the silver halide emulsion of the present invention is a polydisperse silver halide emulsion having cubic grains preferably the compound of formula I is present in the range of from 350mg per mole of Ag to 650mg per mole of Ag.

The silver halide emulsion of the present invention may have been chemically sensitised by any of the well known means for example by use of sulphur, selenium and noble metals. Examples of suitable sensitising compounds are sodium thiosulphate and thiocyanate and mercury, gold, palladium and platinum salts.

The silver halide of the emulsion of the present invention is preferably a predominantly bromide emulsion comprising at least 80% bromide by mole %. A particularly suitable emulsion comprises 94% by mole % bromide and 6% mole % of iodide.

The silver halide emulsion of the present invention may contain any of the additives commonly used in photographic emulsions for example wetting agents, stabilising agents, polyethylene oxides, metal sequestering agents, and growth or crystal habit modifying agents commonly used for silver halide such as adenine.

Preferably the dispersing medium for the silver halide emulsion is gelatin or a mixture of gelatin and a water-soluble latex for example a latex vinyl acrylate-containing polymer. Most preferably if such a latex is present in the final emulsion it is added after all crystal growth has occurred. However, other water-soluble colloids for example casein, polyvinylpyrrolidone or polyvinyl alcohol may be used alone or together with gelatin.

According to another aspect of the present invention, there is provided a photographic material which comprises coated on a photographic base at least one negative working silver halide emulsion which comprises a compound of formula I.

The photographic base may be any of the bases used for photographic film materials, for example transparent bases such as cellulose triacetate, cellulose acetate-butyrate or biaxially orientated polyethylene terephthalate or opaque bases such as baryta coated paper base of polyethylene coated paper base.

The following show the preparation of the compounds of formula I.

PREPARATION I

A. 3-Methyl-2-[(3-methyl-2-thiazolidinylidene)-methyl]-benzoxazolium iodide.

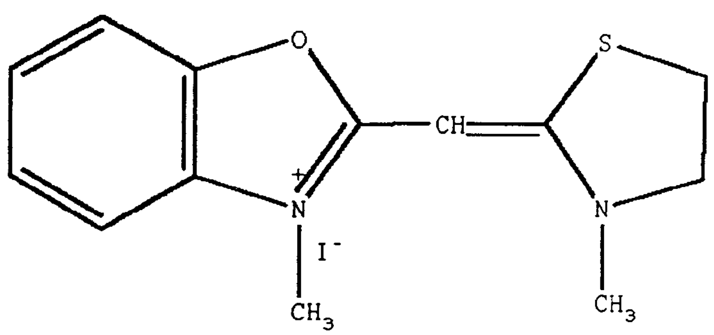

COMPOUND A

A mixture of 2-methylbenzoxazole (13.3g), 4,5-dihydro-2-(methylthio)-thiazole (20.0g), and methyl p-toluene-sulphonate (55.9g) was heated in an oil-bath at 140°C for five hours. Pyridine (100ml) was added and the mixture was refluxed for 30 minutes. The solution was poured, with stirring into a solution of sodium iodide (72g) in water (150ml), and the mixture was cooled. The resulting solid was filtered off and washed thoroughly with water. After drying, it was warmed with toluene and refiltered, and finally recrystallised from methanol.

The product was obtained as pale cream-coloured crystals, mp 311-312°C (decomp).

B. 3-Methyl-2-[3-methyl-2-thiazolidinylidene)-methyl]benzoxazolium perchlorate.

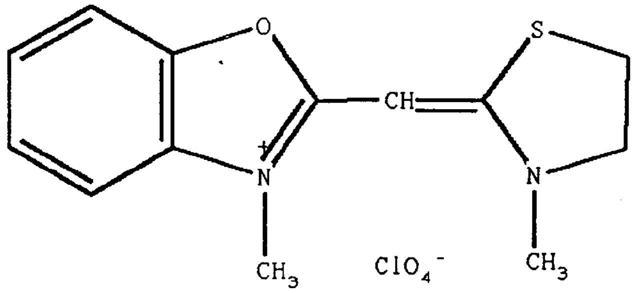

COMPOUND B

Preparation as in A, but by treating the reaction mixture with aqueous sodium perchlorate. solution, the product was obtained as pale-cream coloured crystals, mp 310-312° (decomp) after,crystallisation from pyridine.

PREPARATION 2

3-Methyl-2-[(3-methyl-2-thiazolidinylidene)-methyl]-5-phenylbenzoxazolium iodide.

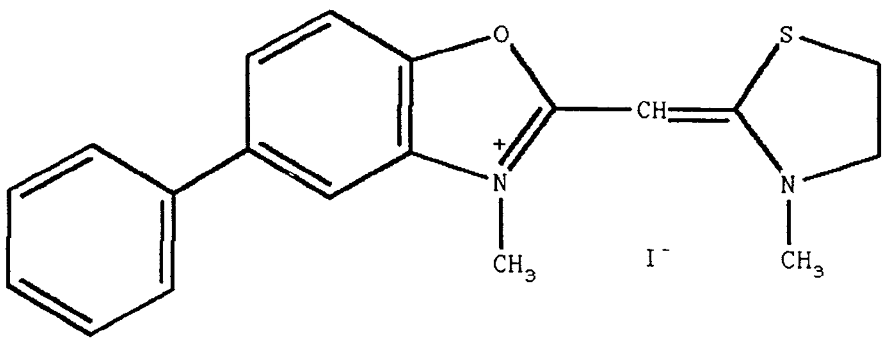

COMPOUND C

A mixture of 2-(methylthio)-5-phenylbenzoxazole (14.5g), 4,5-dihydro-2-methylthiazole (4.75g) and methyl p-toluenesulphonate (28.0g) was heated at 140°C for four hours. Pyridine (50ml) was added and the mixture was refluxed for 40 minutes. The resulting solution was cooled and treated with water (100ml) and the solid which separated was filtered off. The filtrate was treated with sodium iodide (30g) and concentrated hydrochloric acid (50ml). The precipitate was filtered off, washed with water, dried, warmed with toluene and refiltered; it was finally crystallised from methanol to yield the product as pale yellow crystals, mp 262-264°C.

By use of similar methods, the compounds D to K were prepared.

PREPARATION 3

5-chloro-3-methyl-2-[(3-methyl-2-thiazolidinylidene)-methyl}-benzoxazolium bromide.

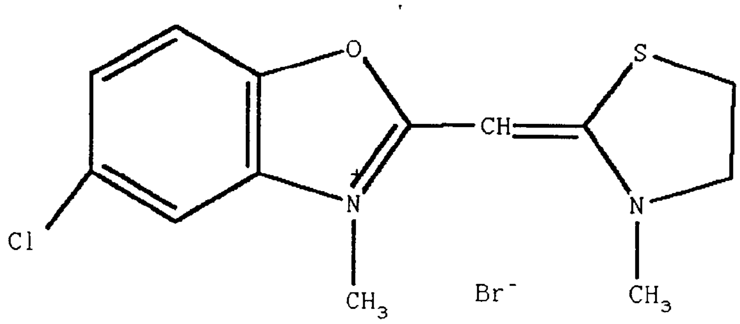

COMPOUND D

The compound was obtained as pale yellow crystals, mp 318-319°C (decomp) by crystallisation from

methanol.

PREPARATION 4

1-Methyl-2-[(3-methyl-2-thiazolidinylidene)-methyl-naphtho [1,2-d] oxazolium iodide.

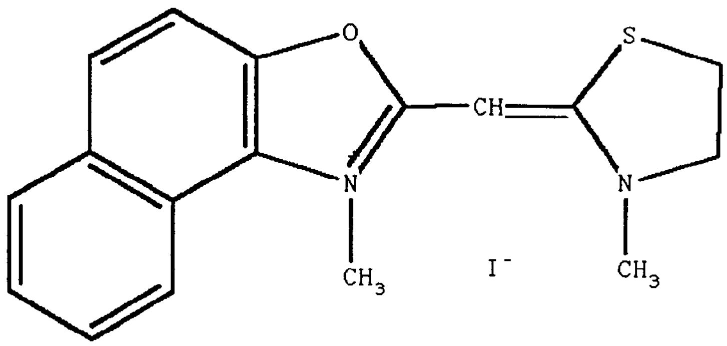

COMPOUND E

The product was obtained as pale yellow crystals, mp 264-265°C (decomp), by crystallisation from methanol.

PREPARATION 5

2,5,6-Trimethyl-2-[(3-methyl-2-thiazolidinylidene)methyl]-benzoxazolium bromide.

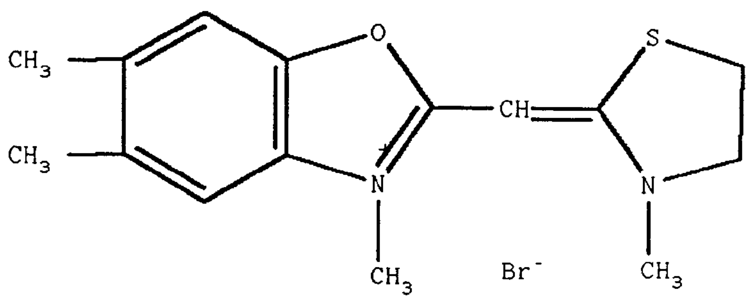

COMPOUND F

After crystallisation from ethanol, the product was obtained as cream-coloured crystals, mp 306-307°C (decomp).

PREPARATION 6

5-Methoxy-3-methyl-2-[(3-methyl-2-thiazolidinylidene)-methyl]-benzoxazolium iodide.

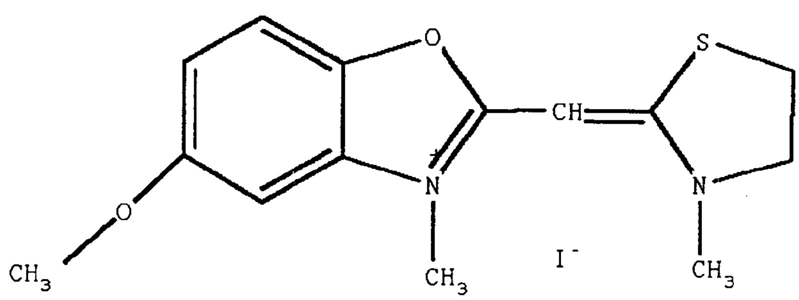

COMPOUND G

The compound was obtained as cream-coloured crystals, mp 284-285°C; by crystallisation from methanol.

PREPARATION 7

2-[(3,5-Dimethyl-2-thiazolidinylidene)-methyl]-3-methylbenzoxazolium iodide.

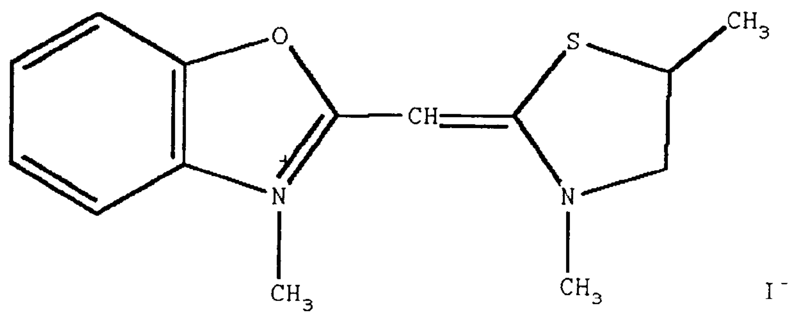

COMPOUND H

The compound was obtained as cream-coloured crystals, mp 278-279°C by crystallisation from methanol.

PREPARATION 8

3-ethyl-2-[(3-ethyl-2-thiazolidinylidene)-methyl]-benzoxazolium iodide.

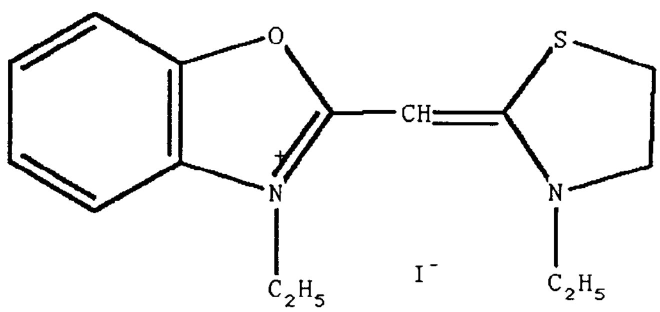

COMPOUND I

The compound was crystallised from methanol, to give cream-coloured crystals, mp 248-250°C.

PREPARATION 9

3-Methyl-2-[(3-methyl-5-phenyl-2-thiazolidinylidene)-methyl]-benzoxazolium iodide.

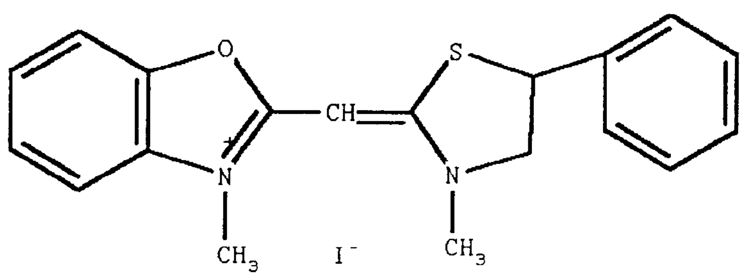

COMPOUND J

After crystallisation from methanol, the product was obtained as pale yellow crystals, mp 260-261°C.

PREPARATION 10

3-Methyl-2-[(3,4,4-trimethyl-2-thiazolidinylidene)-methyl]-benzoxazolium iodide.

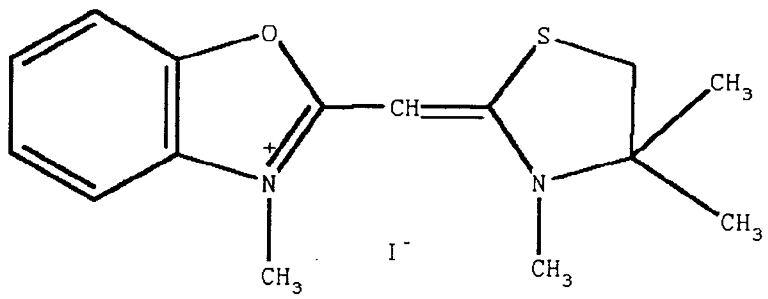

COMPOUND K

After crystallisation from ethanol, the product was obtained as cream-coloured crystals, mp 257-258°C (decomp).

PREPARATION 11

3-(2-Methoxyethyl)-2-[(3-methyl-2-thiazolidinylidene)-methyl]-benzoxazolium iodide.

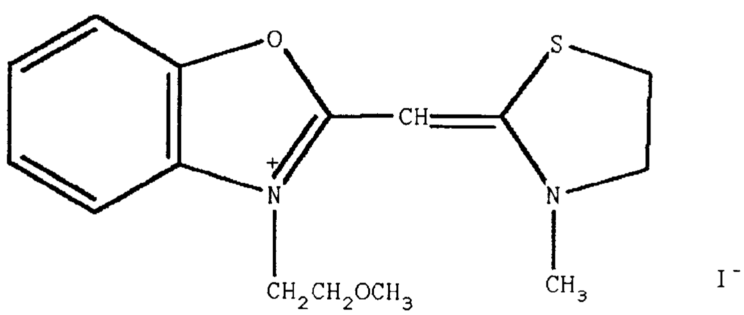

COMPOUND L

Quaternary salts were prepared as follows:-

i) 2-Methylbenzoxazole (6.65g) and 2-methoxyethyl p-toluenesulphonate (12.65g) were heated together at 140°C for 2 hours.

ii) 4,5-Dihydro-2-(methylthio)-thiazole (8.0g) and methyl p-toluenesulphonate (12.3g) were heated together at 100°C for 1.5 hours.

The products were combined, and refluxed for 40 minutes with pyridine (50ml). The resulting solution was poured into a solution of sodium iodide (15g) in water (150ml) and concentrated hydrochloric acid (50ml) was stirred in. The solid, which slowly crystallised out, was filtered off, was washed well with water, then ether and dried. After crystallisation from ethanol, the compound was obtained as pale yellow crystals, mp 223-225°C.

PREPARATION 12

2-[3,(2-methoxyethyl)-2-thiazolidinylidene)-methyl]-3-methylbenzoxazolium tetrafluoro-borate.

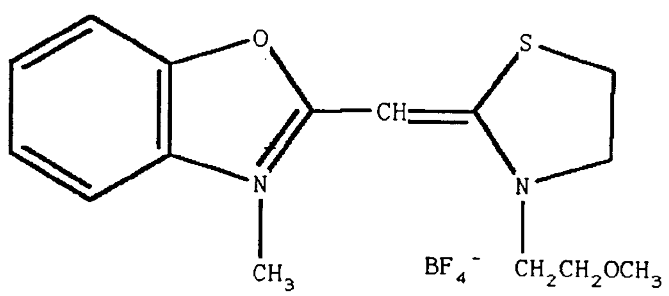

COMPOUND M

The compound was prepared by a method similar to that of Preparation 11; after crystallisation from ethanol, it was obtained as pale yellow crystals, mp 142-144°C.

PREPARATION 13

2,5,7-Trimethyl-2-[(3-methyl-2-thiazolidinylidene)-methyl]-benzoxazolium bromide.

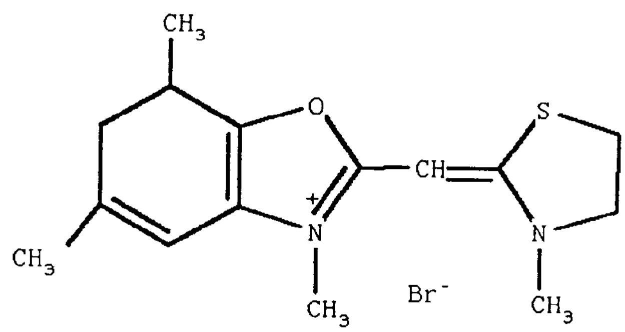

COMPOUND N

By use of a method similar to that of Preparation 1, the compound was obtained as colourless crystals, mp 326-327°C, after crystallisation from methanol.

The compounds used in the following Examples are indicated in the preparations. For example, compound B was prepared in Preparation 1.

EXAMPLE 1

Use with Negative-Working Silver Halide Emulsion Having Tabular Grains

A silver halide emulsion was prepared which comprised silver halide crystals having an aspect ration of 25:1 and a mean grain size of 0.62μm (diameter of sphere of equivalent volume). The core of the crystals were pure bromide but 6 mol % iodide was distributed evenly in the remainder of the crystals. Gelatin was the binder.

Three series of tests using this emulsion were then carried out.

Test 1: Emulsion Not Chemically Sensitised

Compound B 267mg per mole of silver in methanol was added to the emulsion at 40°C. After 30 minutes the emulsion was coated on subbed polyester base to give a silver coating weight of $50mg/dm^2$ and a gelatin coating weight of $97mg/dm^2$. After the coating had dried it was cut into strips.

The strips were light exposed to 250 Lux for a range of exposure times and developed in a developer I of the formula:-

| | |
|---|---|
| Metol | 2 g |
| Hydroquinone | 5 g |
| Sodium Sulphite | 100 g |
| Borax | 3 g |
| Sodium Tripolyphosphate | 3.5g |
| Water to | 1 Litre |

for eight minutes at 20°C

The results are shown in Table 1. The foot speed (0.1 density units above fog, $S_{0.1}$) being shown for a range of exposure times.

TABLE 1

| | EXPOSURE TIME IN SECONDS | | |
|---|---|---|---|
| Coating | t = $10^{-3}$s | t = 1s | t = 60s |
| With Compound B ($S_{0.1}$) | 3.39 | 3.27 | 2.97 |
| Without Compound B ($S_{0.1}$) | 3.12 | 3.06 | 1.99 |

This shows that Compound B decreases low intensity reciprocity failure. The speed decrease at long exposure times is decreased by the presence of Compound B.

Test 2: Emulsion Chemically Sensitised

Samples of the above tabular emulsion were optimally chemically sensitised by being digested at 52°C with sulphur and gold in the presence of sodium thiocyanate. The emulsion was then stabilised by addition of a solution of 4-hydroxy-b-methyl-1,3,3a,7-tetraazaindene stabiliser. After 30 minutes various amounts of Compounds B, C and E were then added as methanolic solutions to individual portions of the above emulsion.

All the emulsions were then coated on subbed polyester at a silver coating weight of 50mg/$dm^2$ and a gelatin coating weight of 97mg/$dm^2$.

After the coating had dried they were cut into strips exposed to light (1/50, 250 Lux) and developed in Developer I (8 minutes).

Table 2 shows the foot speed measured when B, C, D and E were present in the emulsion at a range of compound concentrations.

TABLE 2

| CONCENTRATION (mg per mol Ag) | $S_{0.1}$ | | | |
|---|---|---|---|---|
| | Cpd B | Cpd C | Cpd D | Cpd E |
| 0 | 4.37 | 4.43 | 4.43 | 4.43 |
| 133 | 4.55 | 4.44 | 4.48 | 4.48 |
| 267 | 4.58 | 4.49 | 4.51 | 4.48 |
| 400 | 4.61 | 4.52 | 4.62 | 4.52 |

The results demonstrate that between 0.1-0.2 logE, speed increases over an optimally chemically sensitised emulsion on addition of compounds of formula I were obtained.

Test 3: Emulsion Optimally Chemically Sensitised and Panchromatically Sensitised

To the chemically sensitised emulsion as just prepared there was added a fixed amount (200mg per mole of silver) of Compound B and varying amounts of the panchromatic sensitising dye W of the formula:-

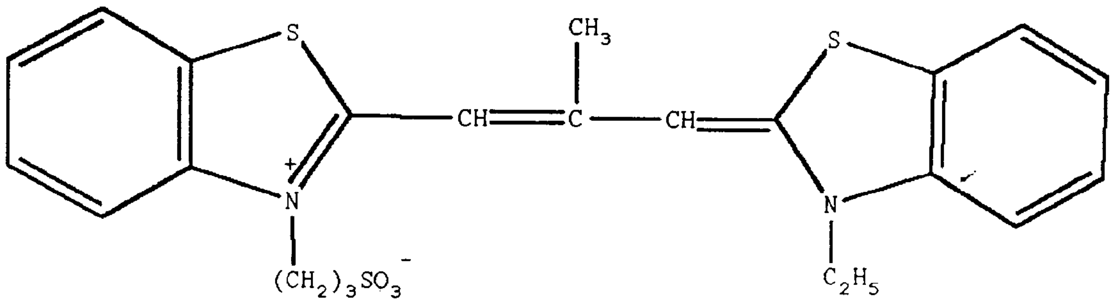

DYE W

To another set of emulsions there was added the same amounts of dye W in the absence of Compound B.

All the emulsions were then coated on subbed polyester base at a silver coating weight of 50mg/dm$^2$ and a gelatin coating weight of 97mg/dm$^2$. After drying the strips were prepared from the coated material. One set of strips were then subjected to an overall 250 Lux, white light exposure of 1/50 second then developed for 8 minutes at 20°C in the developer I. A second set of strips were then subjected to blue, green and red exposures (1/30s, 250 Lux) respectively and were developed in developer II (10 minutes) of formula:-

| | |
|---|---|
| Metol | 2 g |
| Hydroquinone | 8 g |
| Sodium Sulphite | 90 g |
| Sodium Carbonate | 45 g |
| Potassium Bromide | 5 g |
| Water to | 1 Litre |

The results are shown in Tables 3 and 4.

TABLE 3

| Dye W (mg per mol of Ag) | $S_{0.1}$ With Compound B | Without Compound B |
|---|---|---|
| 0 | 4.56 | 4.54 |
| 67 | 4.90 | 4.71 |
| 133 | 5.14 | 4.86 |
| 200 | 5.14 | 4.91 |
| 267 | 5.16 | 4.92 |
| 383 | 5.11 | 5.00 |

These tables show that the presence of Compound B increases the sensitivity of the emulsion over and above the sensitivity due to dye W alone.

TABLE 4

| Dye W (mg per mole Ag | Blue Speed ($S_{0.1}$) With Cpd B | Blue Speed ($S_{0.1}$) Without Cpd B | Green Speed ($S_{0.1}$) With Cpd B | Green Speed ($S_{0.1}$) Without Cpd B | Red Speed ($S_{0.1}$) With Cpd B | Red Speed ($S_{0.1}$) Without Cpd B |
|---|---|---|---|---|---|---|
| 133 | 4.13 | 4.08 | 4.45 | 4.21 | 4.47 | 4.21 |
| 267 | 4.18 | 4.13 | 4.59 | 4.38 | 4.58 | 4.40 |

EXAMPLE 2

Negative-Working Silver Halide Emulsion Having Cubic Habit Grains

A polydisperse silver iodobromide emulsion containing 2.6 mol % iodide and having a median grain size of 0.48μm was prepared. After the emulsion had been optimally chemically sensitised using sulphur and gold, then 350mg of Compound B in methanol was added per mol of Ag on the emulsion. The emulsion was then stabilised using a tetraazaindolizine stabiliser and then coated on subbed polyester base at a coating weight of 1.54g Ag/m$^2$ and gelatin coating weight of 1.38g/m$^2$.

For comparison a similar emulsion was prepared and coated but without adding Compound B. After exposure to blue light (200 Lux, 1 second) the following sensitometric parameters were measured after development in Developer III for 8 minutes at 20°C.

The formula of Developer III is as follows:-

| | |
|---|---|
| 1-Phenyl-3-Pyrazolidinone | 1 g/l |
| Hydroquinone | 12 g/l |
| $K_2SO_3$ | 19.9g/l |
| $Na_2SO_3$ | 38 g/l |
| $Li_2SO_3$ | 0.6g/l |
| $K_2CO_3$ | 19.6g/l |
| $KHCO_3$ | 13.3g/l |
| KBr | 1.5g/l |
| Benzotriazole | 0.5g/l |
| $Na_4EDTA\ 2H_2O$ | 4.0g/l |
| $KCH_3COO$ | 0.7g/l |
| Ethylidiglycol | 50 g/l |
| Water to | 1 Litre |

The results are shown in Table 5.

TABLE 5

| COATING | MAX DENSITY | E1 | E2 | MIN DENSITY |
|---|---|---|---|---|
| Without Cpd B | 1.06 | 5.97 | 5.17 | 0.04 |
| With Cpd B | 1.23 | 6.27 | 5.49 | 0.05 |

E1 = 6-log exposure necessary to attain a density of 5% between Dmax and Dmin.
E2 = 6-log exposure necessary to attain an optimal density of 50% between Dmax and Dmin.

This shows that Compound B of formula I increases the sensitivity of the emulsion over and above the natural sensitivity of the emulsion to blue light.

## EXAMPLE 3

Negative Working Silver Halide Emulsion Having Cubic Habit Grains

A polydisperse silver iodobromide emulsion containing 5.2 mol % iodide and having a median grain size of 0.36um was prepared. After the emulsion had been optimally chemically sensitised using sulphur and gold, 132g per mole of Ag of the stabiliser of the formula:-

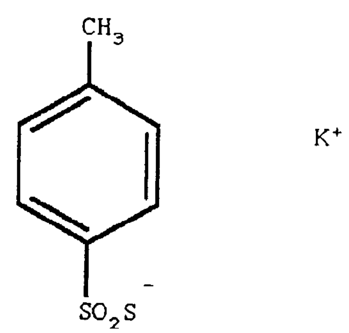

was added with stirring for 2 minutes. Then 640mg per mole of Ag of Compound B and 289mg of the green sensitising dye Y per mole of silver were added to the emulsion as a methanolic solution.

The formula of dye Y is:-

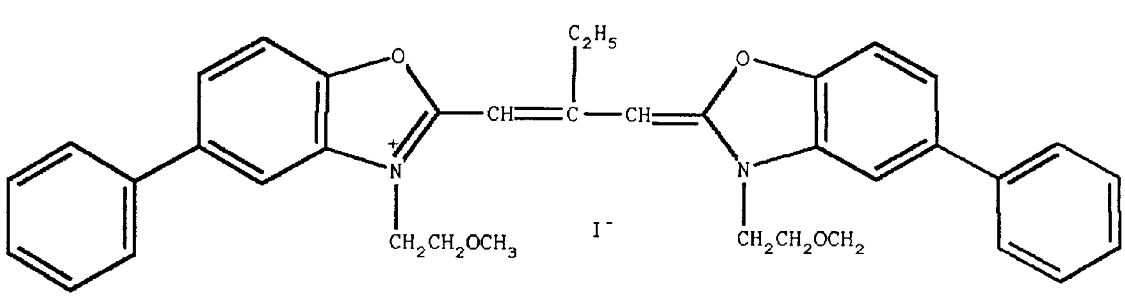

The emulsion was then coated on subbed polyester base at a silver coating weight of 1.41g/m$^2$ and a gelatin coating weight of 1. 75g/m$^2$.

A similar coating was prepared which contained the above amount of dye Y but no Compound B.

Both coatings were then subjected to 200 Lux exposure to green light for three different exposure times

(t). The coatings were then developed in Developer III for 8 minutes at 20°C.

The following results were obtained:

TABLE 6

| Coating | Max Density | Min Density | E2 t=1s | E2 t=512s | E2 t=1024s |
|---|---|---|---|---|---|
| With Cpd B and Dye Y | 1.31 | 0.10 | 5.81 | 5.18 | 5.13 |
| With only Dye Y | 1.33 | 0.08 | 5.81 | 5.04 | 4.96 |

This shows that the presence of Compound B increases green speed at long exposure times (t) by decreasing low intensity reciprocity failure.

## Claims

1. A negative-working silver halide emulsion which is characterised in that it comprises a monomethine compound of the general formula I:-

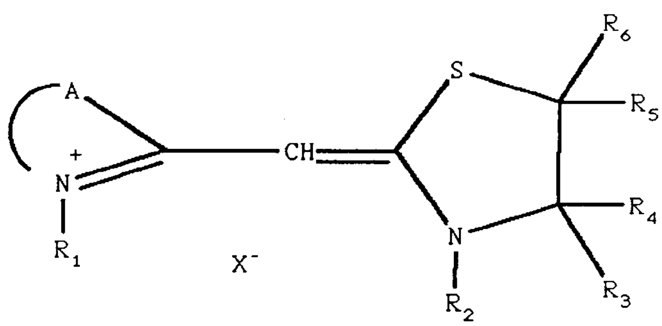

....I

where A represents the atoms necessary to complete a benzoxazole or naphthoxazole ring which is optionally substituted, $R_1$ and $R_2$ are each alkyl, hydroxyalkyl or alkoxy alkyl groups wherein the alkyl moiety comprises 1 to 4 carbon atoms, $R_3$, $R_4$, $R_5$ and $R_5$ are each hydrogen atoms or alkyl groups having 1 to 4 carbon atoms or phenyl groups, and X is an anion.

2. A negative-working silver halide emulsion according to Claim 1 characterised in that A in formula I represents the atoms necessary to complete a benzoxazole or naphthoxazole ring which is unsubstituted or substituted by alkyl or alkoxy each having 1 to 4 carbon atoms, halogen or phenyl, $R_1$ and $R_2$ are each alkyl or alkoxy alkyl wherein the alkyl moiety comprises 1 to 4 carbon atoms, $R_3$, $R_4$, $R_5$ and $R_6$ are each hydrogen, alkyl having 1 to 4 carbon atoms or phenyl and $X^-$ is $ClO_4^-$, $BF_4^-$ or a halide.

3. A negative working silver halide emulsion according to Claim 2 characterised is that in formula I, both $R_1$ and $R_2$ are methyl groups.

4. A negative working silver halide emulsion which is characterised in that it comprises a monomethine compound of one of the following formulae A to N:-

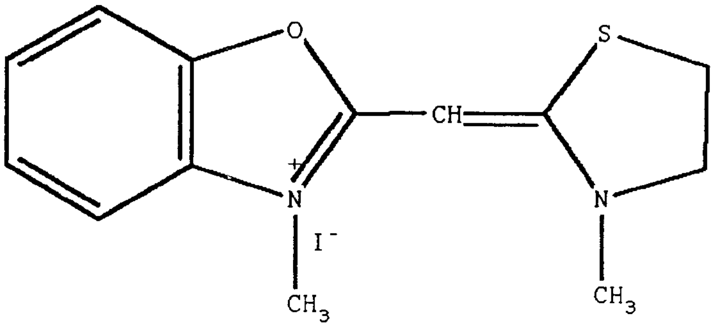

COMPOUND A

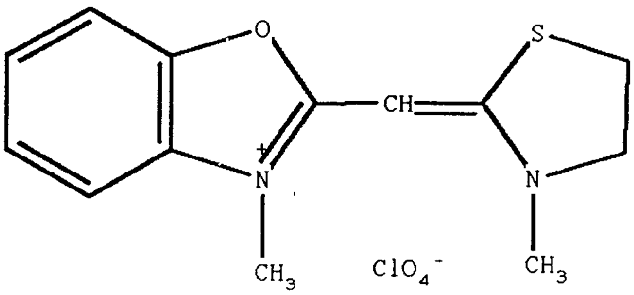

COMPOUND B

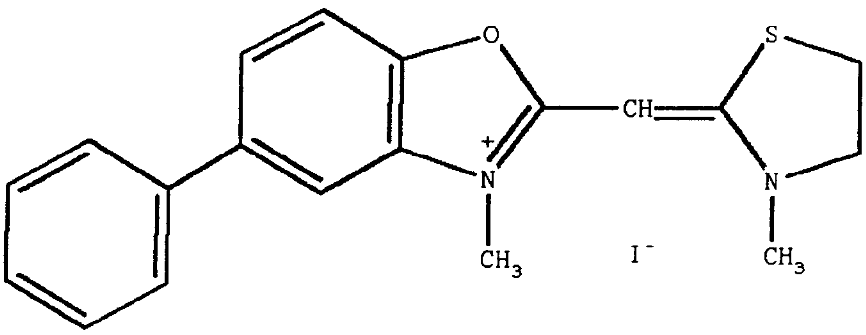

COMPOUND C

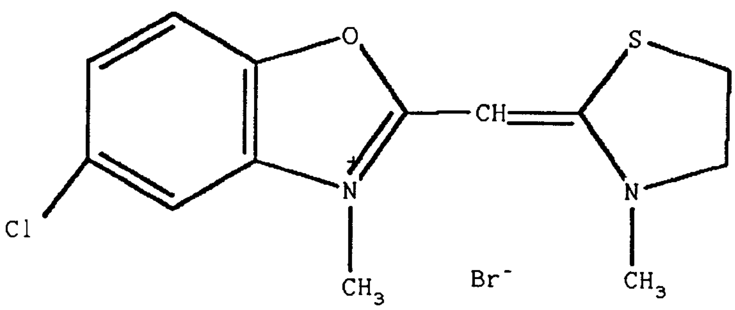

COMPOUND D

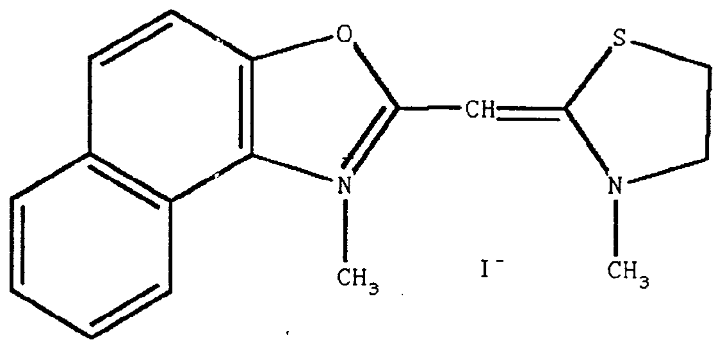

COMPOUND E

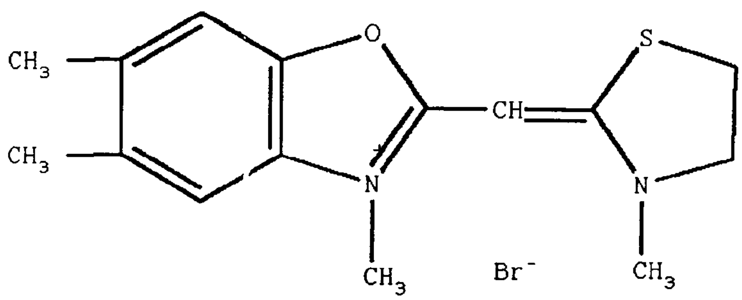

COMPOUND F

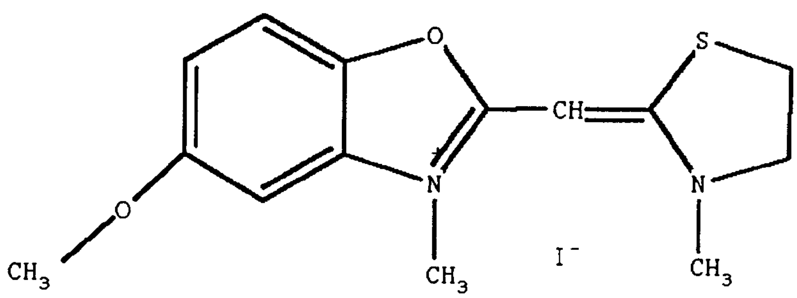

COMPOUND G

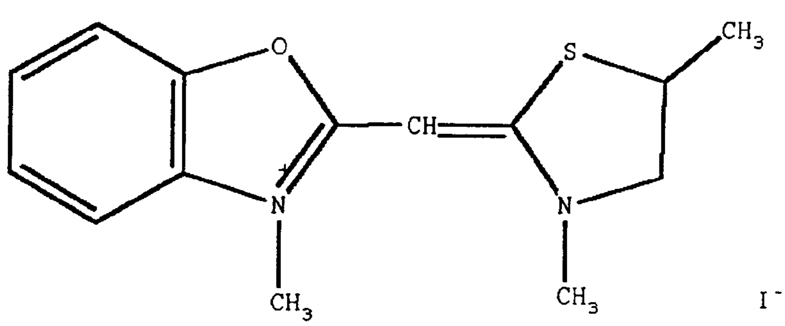

COMPOUND H

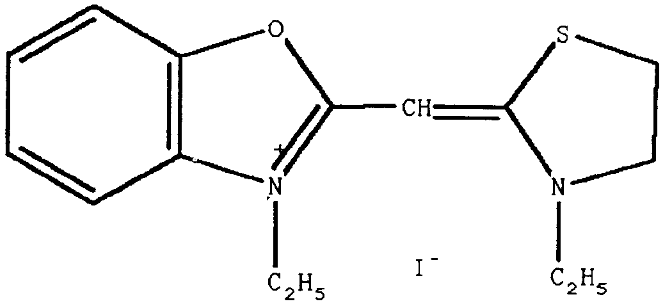

COMPOUND I

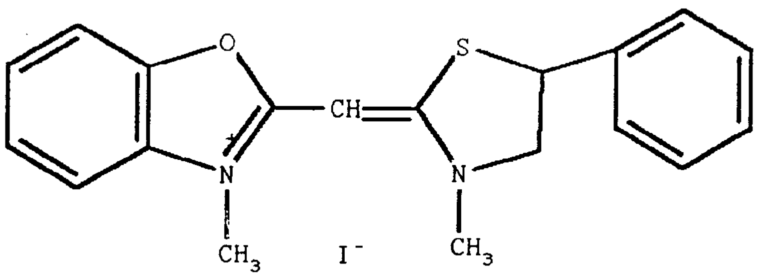

COMPOUND J

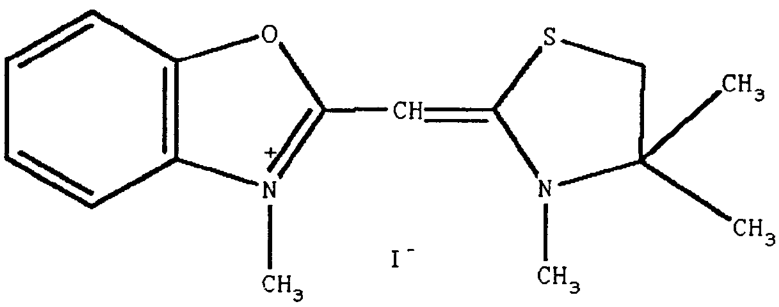

COMPOUND K

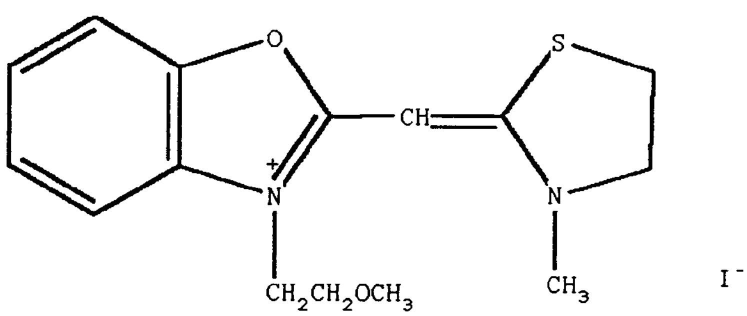

COMPOUND L

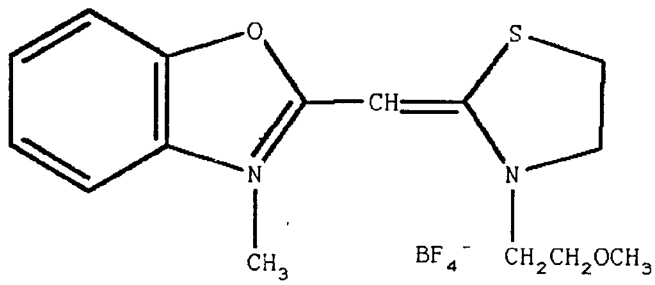

COMPOUND M

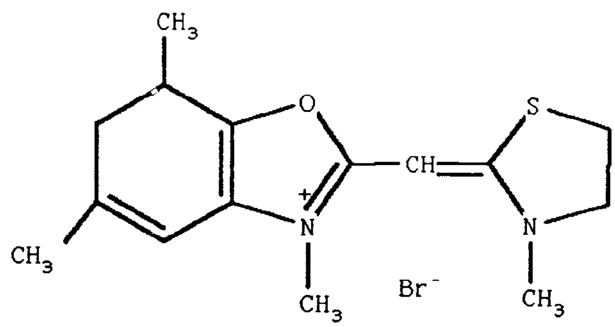

COMPOUND N

5. A photographic silver halide emulsion according to Claim 1 which is characterised in that the amount of monomethine compound in formula I is from 50mg per mole of silver to 700mg per mole of silver.

6. A photographic silver halide emulsion according to Claim 1 characterised in that the silver halide grains are of the tabular habit having an aspect ratio of greater than 5:1.

7. A photographic silver halide emulsion according to Claim 6 characterised in that the amount of monomethine compound of Formula I is from 100mg per mole of silver to 400mg per mole of silver.

8. A photographic silver halide emulsion according to Claim 1 characterised in that the silver halide grains are polydisperse having a cubic habit.

9. A photographic silver halide emulsion according to Claim 8 characterised in that the amount of monomethine compound of Formula I is from 350mg per mole of silver to 650mg per mole of silver.

10. A photographic silver halide material which is characterised in that it comprises at least one silver halide emulsion layer as claimed in Claim 1.

## Patentansprüche

1. Negativ-arbeitende Silberhalogenidemulsion, dadurch gekennzeichnet, daß sie eine Monomethinverbindung der allgemeinen Formel I enthält:

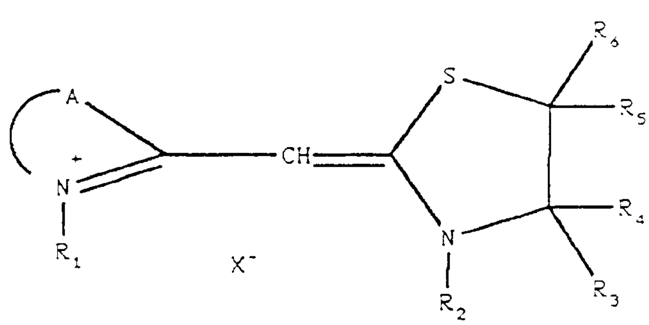

worin A die zur Ergänzung zu einem gegebenenfalls substituierten Benzoxazol- oder Naphthoxazolring erforderlichen Atome darstellt und $R_1$ und $R_2$ je für Alkyl-, Hydroxyalkyl- oder Alkoxyalkylgruppen mit 1 bis 4 Kohlenstoffatomen im Alkylteil, $R_3$, $R_4$, $R_5$ und $R_6$ je für Wasserstoffatome, Alkylgruppen mit 1 bis 4 Kohlenstoffatomen oder Phenylgruppen und X für ein Anion stehen.

2. Negativ-arbeitende Silberhalogenidemulsion nach Anspruch 1, dadurch gekennzeichnet, daß A in Formel I die zur Ergänzung zu einem gegebenenfalls mit Alkyl oder Alkoxy mit je 1 bis 4 Kohlenstoffatomen, Halogen oder Phenyl substituierten Benzoxazol- oder Naphthoxazolring erforderlichen Atome darstellt und $R_1$ und $R_2$ je für Alkyl oder Alkoxyalkyl mit 1 bis 4 Kohlenstoffatomen im Alkylteil, $R_3$, $R_4$, $R_6$ und $R_6$ je für Wasserstoff, Alkyl mit 1 bis 4 Kohlenstoffatomen oder Phenyl und $X^-$ für $ClO_4^-$, $BF_4^-$ oder ein Halogenid stehen.

3. Negativ-arbeitende Silberhalogenidemulsion nach Anspruch 2, dadurch gekennzeichnet, daß in Formel I sowohl $R_1$ als auch $R_2$ für Methylgruppen stehen.

4. Negativ-arbeitende Silberhalogenidemulsion, dadurch gekennzeichnet, daß sie eine Monomethinverbindung einer der nachfolgenden Formeln A bis N enthält:

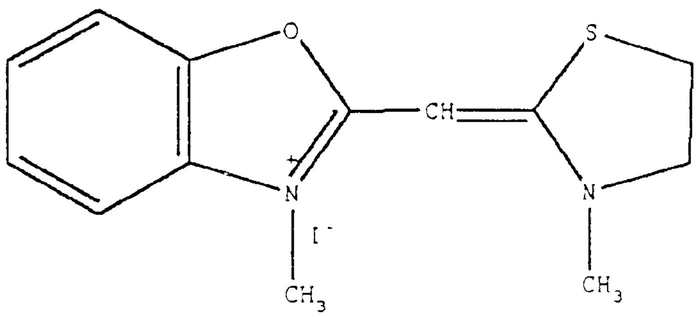

Verbindung A

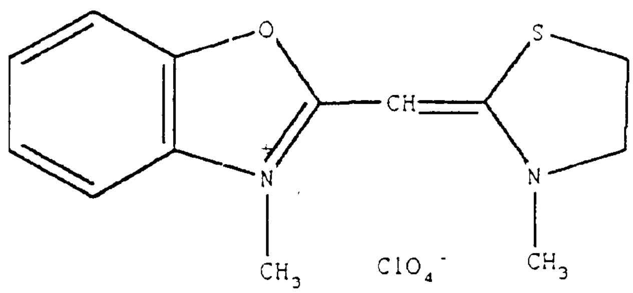

Verbindung B

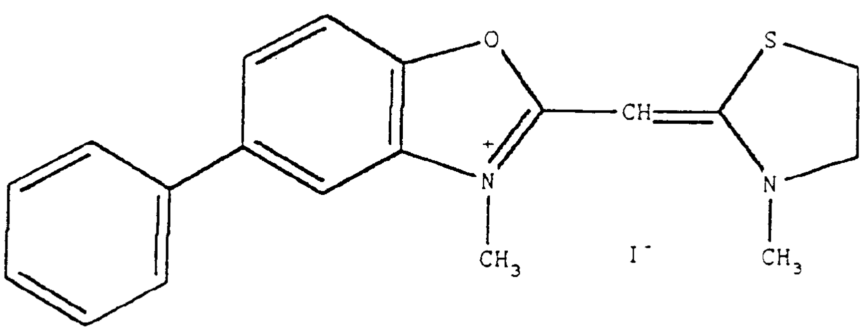

Verbindung C

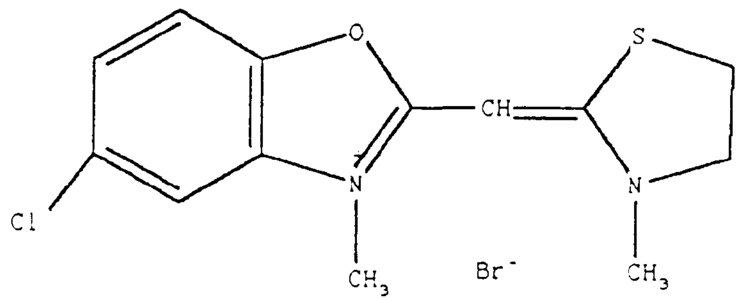

Verbindung D

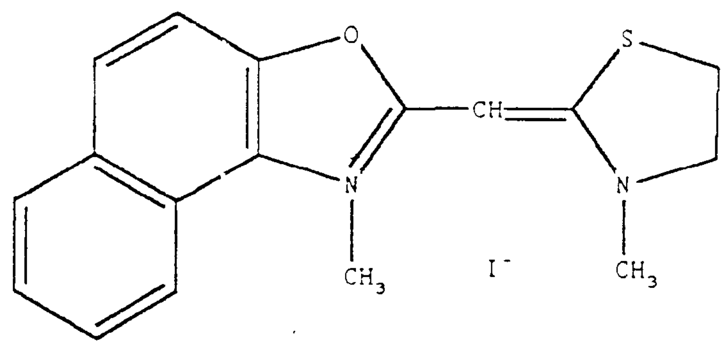

Verbindung E

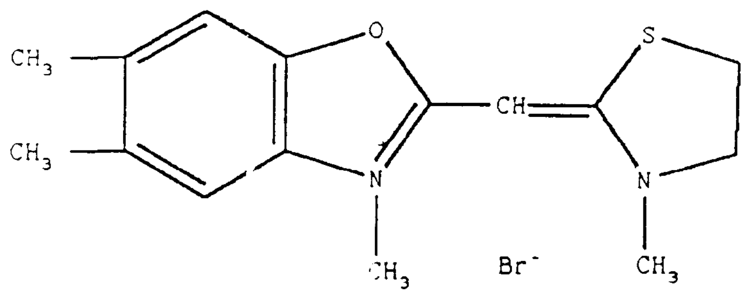

Verbindung F

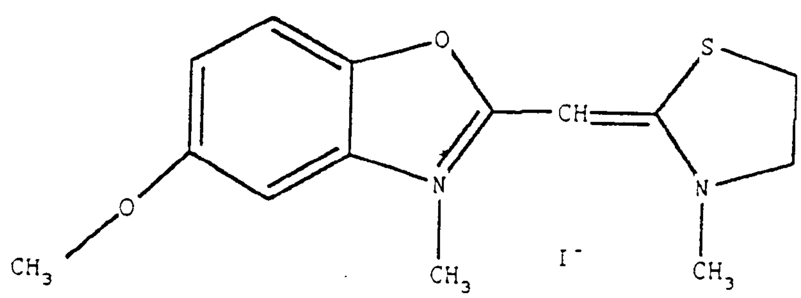

Verbindung G

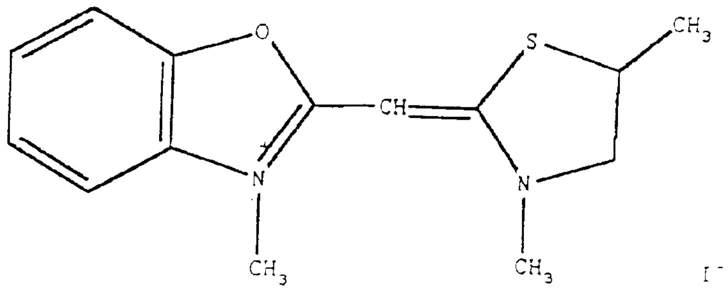

Verbindung H

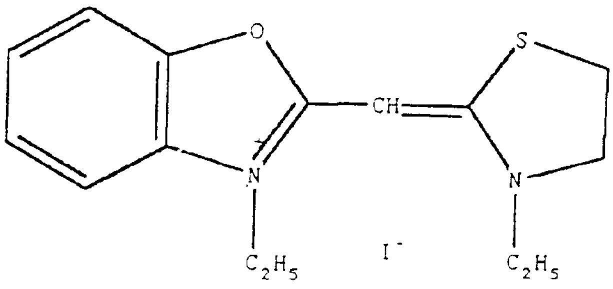

Verbindung I

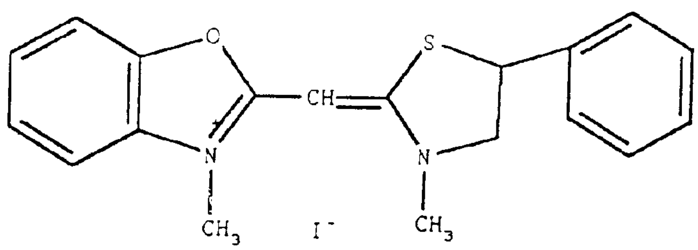

Verbindung J

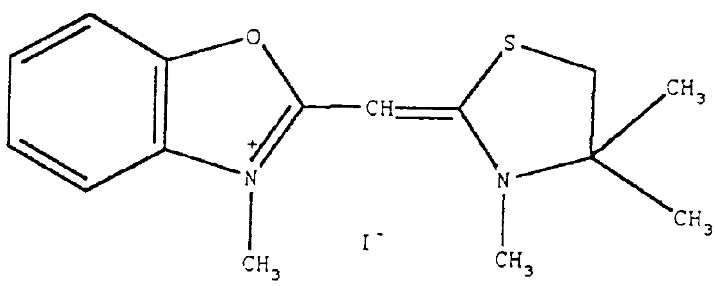

Verbindung K

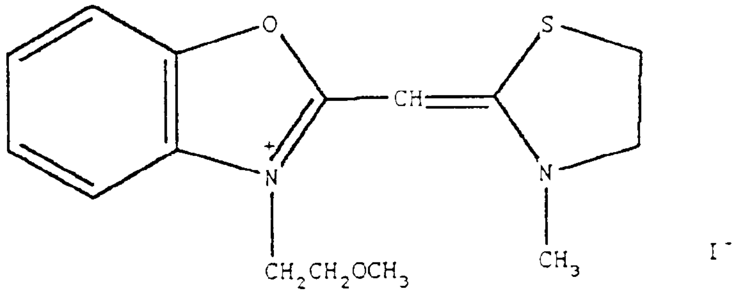

Verbindung L

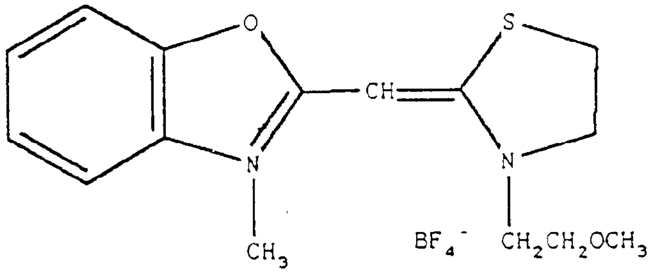

Verbindung M

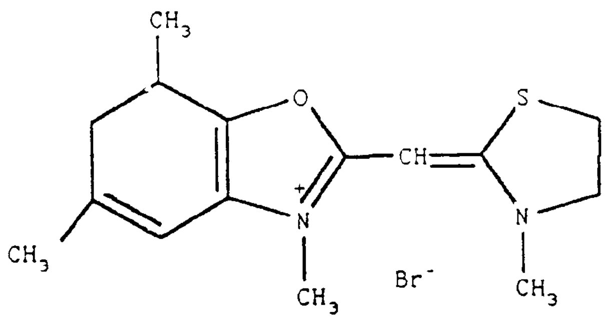

Verbindung N

5. Photographische Silberhalogenidemulsion nach Anspruch 1, dadurch gekennzeichnet, daß die Menge an Monomethinverbindung der Formel I 50 mg pro Mol Silber bis 700 mg pro Mol Silber beträgt.

6. Photographische Silberhalogenidemulsion nach Anspruch 1, dadurch gekennzeichnet, daß die Silberhalogenidkörner einen tafelförmigen Habitus mit einem Längenverhältnis grösser als 5:1 aufweisen.

7. Photographische Silberhalogenidemulsion nach Anspruch 6, dadurch gekennzeichnet, daß die Menge an Monomethinverbindung der Formel I 100 mg pro Mol Silber bis 400 mg pro Mol Silber beträgt.

8. Photographische Silberhalogenidemulsion nach Anspruch 1, dadurch gekennzeichnet, daß die Silberhalogenidkörner polydispers mit einem kubischen Habitus sind.

9. Photographische Silberhalogenidemulsion nach Anspruch 8, dadurch gekennzeichnet, daß die Menge an Monomethinverbindung der Formel I 350 mg pro Mol Silber bis 650 mg pro Mol Silber beträgt.

10. Photographisches Silberhalogenidmaterial,dadurch gekennzeichnet, daß es mindestens eine Silberhalogenidemulsionsschicht nach Anspruch 1 enthält.

## Revendications

1. Une émulsion d'halogénure d'argent pour copiage par inversion, caractérisée en ce qu'elle comprend un composé monométhinique de formule générale I :

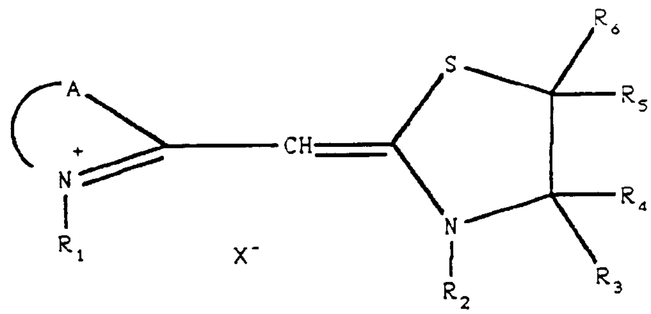

dans laquelle A représente les atomes nécessaires pour compléter un cycle benzoxazole ou naphtoxazole qui est peut être substitué ou non, $R_1$ et $R_2$ sont chacun des groupes alkyle, hydroxyalkyle ou alcoxyalkyle, dans lesquels la partie alkyle comprend 1 à 4 atomes de carbone, $R_3$, $R_4$, $R_5$ et $R_6$ sont chacun des atomes d'hydrogène ou des groupes alkyle ayant 1 à 4 atomes de carbone ou des groupes phényle, et X est un anion.

2. Une émulsion d'halogénure d'argent pour copiage par inversion selon la revendication 1, caractérisée en ce que A dans la formule I représente les atomes nécessaires pour compléter un cycle benzoxazole ou naphtoxazole qui est non substitué ou substitué par des groupes alkyle ou alcoxy ayant chacun 1 à 4 atomes de carbone, un halogène ou un phényle, $R_1$ et $R_2$ sont chacun un alkyle ou alcoxyalkyle dans lesquels la partie alkyle comprend 1 à 4 atomes de carbone, $R_3$, $R_4$, $R_6$ et $R_6$ sont chacun un hydrogène, un alkyle ayant 1 à 4 atomes de carbone ou un phényle et $X^-$ est $ClO_4^-$, $BF_4^-$ ou un halogénure.

3. Une émulsion d'halogénure d'argent pour copiage par inversion selon la revendication 2, caractérisée en ce que, dans la formule I, $R_1$ et $R_2$ sont tous deux des groupes méthyle.

4. Une émulsion d'halogénure d'argent pour copiage par inversion caractérisée en ce qu'elle comprend un composé monométhinique de l'une des formules suivantes A à N :

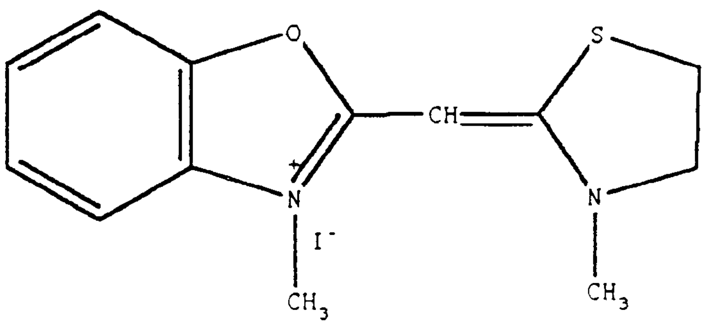

COMPOSE A

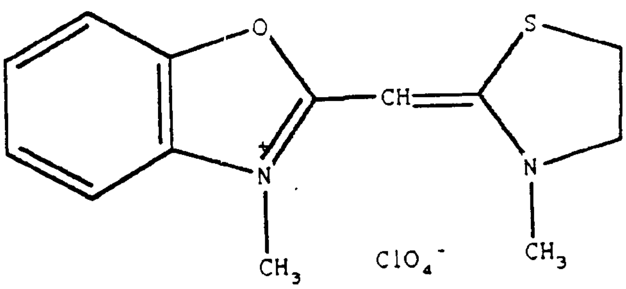

COMPOSE B

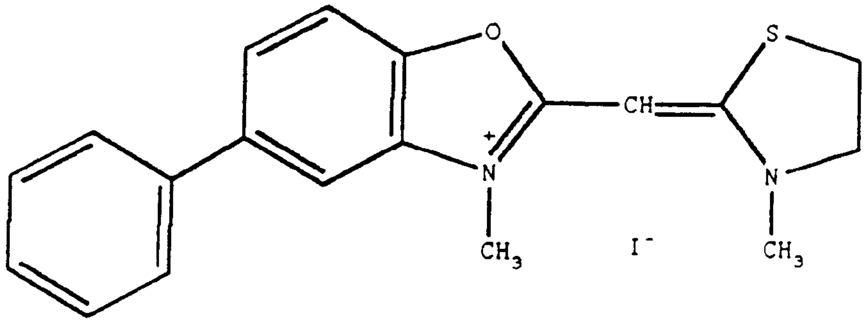

COMPOSE C

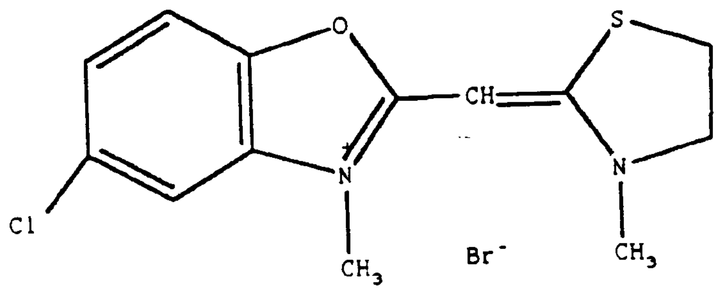

COMPOSE D

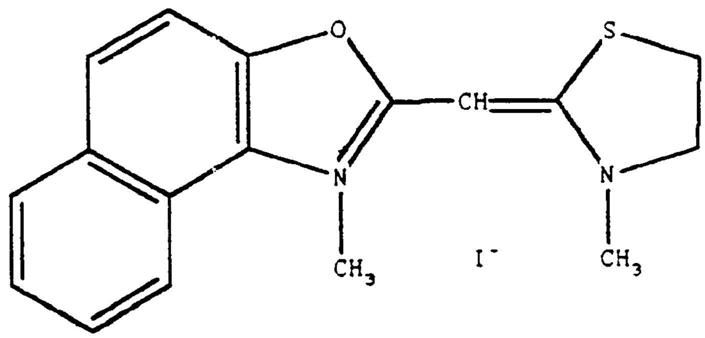

COMPOSE E

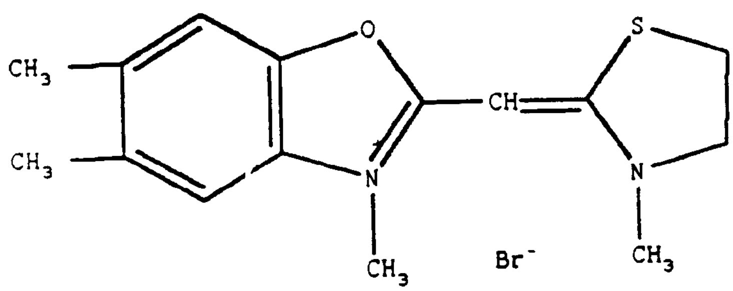

COMPOSE F

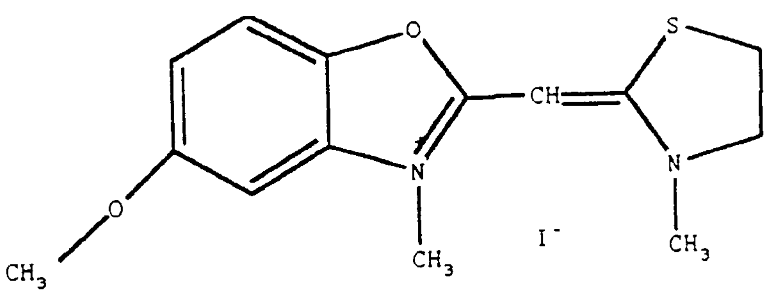

COMPOSE G

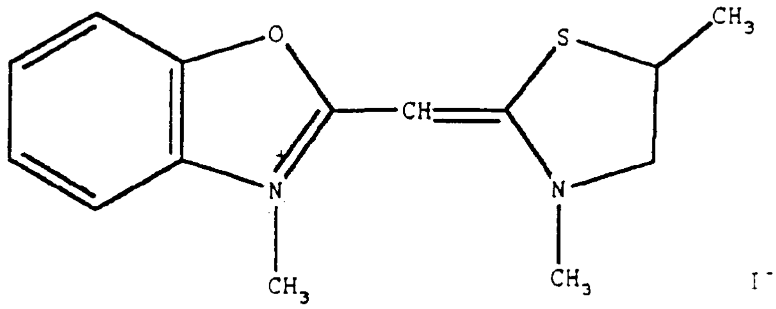

COMPOSE H

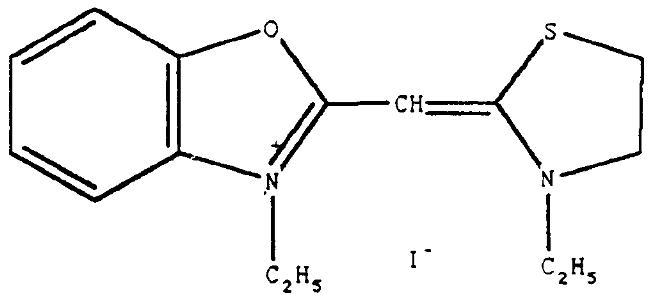

COMPOSE I

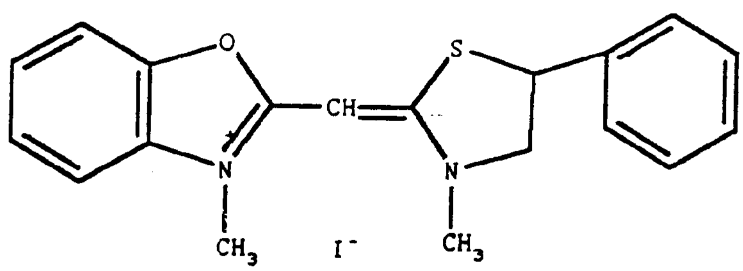

COMPOSE J

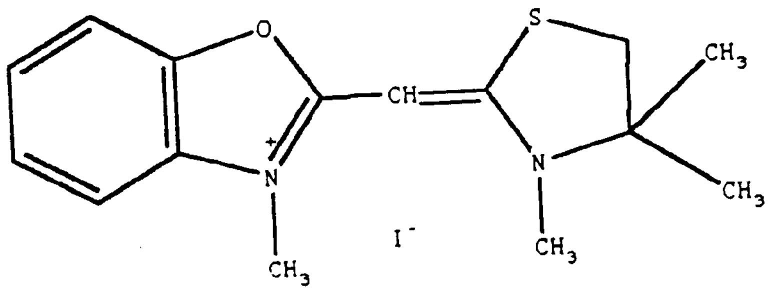

COMPOSE K

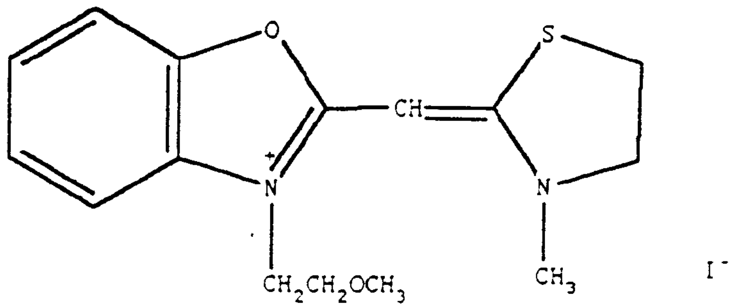

COMPOSE L

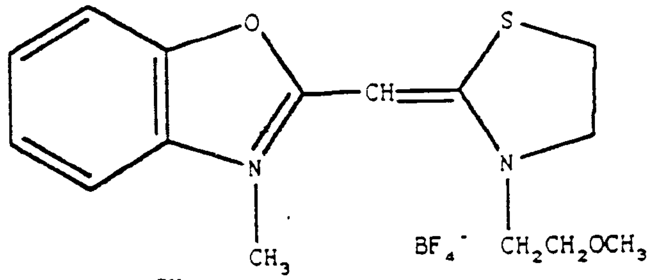

COMPOSE M

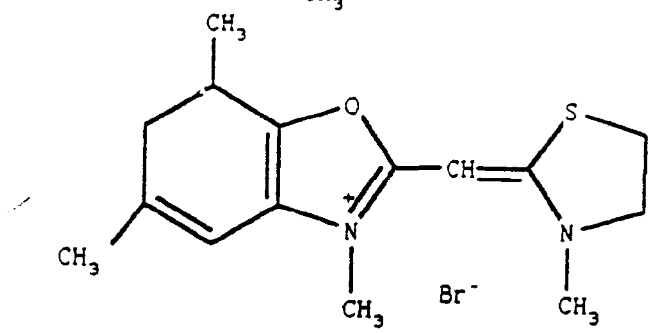

COMPOSE N

5. Une émulsion photographique d'halogénure d'argent selon la revendication 1, caractérisée en ce que la quantité du composé monométhinique, dans la Formule I, est de 50mg par mole d'argent jusqu'à 700mg par mole d'argent.

6. Une émulsion photographique d'halogénure d'argent selon la revendication 1, caractérisée en ce que les grains d'halogénure d'argent sont de structure tabulaire ayant un rapport d'aspect plus grand que 5/1.

7. Une émulsion photographique d'halogénure d'argent selon la revendication 6, caractérisée en ce que la quantité du composé monométhinique de la Formule I est de 100mg par mole d'argent jusqu'à 400mg par mole d'argent.

8. Une émulsion photographique d'halogénure d'argent selon la revendication 1, caractérisée en ce que les grains d'halogénure d'argent sont polydispersés en ayant une structure cubique.

9. Une émulsion photographique d'halogénure d'argent selon la revendication 8, caractérisée en ce que la quantité du composé monométhinique de la Formule I est de 350mg par mole d'argent jusqu'à 650mg par mole d'argent. 10. Une substance photographique d'halogénure d'argent caractérisée en ce qu'elle comprend au moins une couche d'émulsion d'halogénure d'argent selon la revendication 1.